# EUROPEAN PATENT APPLICATION

(11) **EP 4 714 396 A1**
(43) Date of publication of application: **25.03.2026**
(21) Application number: 24202099.8
(22) Date of filing: 24.09.2024
(51) Int. Cl.: A61F 2/04, B29C 43/02, B29D 23/00

(54) **DEVICES AND METHODS ASSOCIATED WITH A TUBE SUITABLE FOR MEDICAL USE**

(71) Applicant: Obducat AB, 22363 Lund (SE)
(72) Inventor: THULIN, Kristian, 22363 Lund (SE); USMAN, Khurram, 22363 Lund (SE); GENEVSKIY, Vladislav, 22363 Lund (SE); SHLEEV, Sergey, 22363 Lund (SE)
(74) Representative: Neij & Lindberg AB

(57) **Abstract**

A tube (100) suitable for medical use, comprising two semi-cylindrical gutter-shaped tube portions (101, 102, of a thermoplastic material, engaged at a joint (110) to form the tube, wherein the tube comprises a nanostructure pattern on an inner surface (103) of the tube, imprinted in each tube portion.

## Description

### Technical field

This disclosure relates to the field of tubes suitable for use in medical treatment, such as catheters, as well as to methods and devices for production of such a tube. Specifically, the proposed solution provides a tube which is resilient towards biofilm growth, by means of nanoimprint on a surface of the tube.

### Background

Nosocomial pathogens-induced infections are one of the major challenges to human health when combined with the threat of antimicrobial resistance (AMR). Adhesive susceptibility of tenacious bacterial communities on indwelling devices is known to be one of the main leading causes of infection in hospital environments. Biofilm growth is associated with various microbial infections like urinary tract infections (UTI), sinusitis, cystic fibrosis, periodontal disease, and chronic wounds, involving prevalent species found in healthcare settings such as E. coli, S. aureus, and P. aeruginosa. Catheter-based UTIs are the leading cause of infection in hospitalized personals.

Existing methods to avoid bacterial biofilms involve repelling and killing. Repelling strategy involves the development of bacteriostatic surfaces employing hydrophobic, slippery and lubricant-based surfaces for prevention of bacterial biofilms. Bactericidal surfaces, on the other hand, are configured to kill bacteria to prevent their growth onto the surface. Such strategies comprise technologies based on positively charged polymer coatings, silver and copper ions, antibiotic release, antimicrobial peptides and enzymes.

However, pathogens persist and remain viable after adherence to a surface. Regardless of their broad application coatings have considerable drawbacks. These disadvantages include cytotoxicity, limited stability, and lack of control over drug release. Conventional methods for solving device-based infection require antibiotic or antimicrobial drug delivery, through the implant or directly to the patient, but they frequently contribute to side effects including overuse and antimicrobial resistance.

### Summary

The proposed solution aims at providing a tube suitable for medical treatment purposes, which is configured to counteract negative side effect caused by insertion and use of the tube inside the body of a human or animal. The proposed solution is defined by the terms of the independent claims.

According to a first aspect, the proposed solution provides a tube suitable for medical use, comprising two semi-cylindrical gutter-shaped tube portions of a thermoplastic material, engaged at a joint to form the tube, wherein the tube comprises a nanostructure pattern on an inner surface of the tube, imprinted in each tube portion.

According to another aspect, the proposed solution provides a method for producing a tube suitable for medical use, wherein the method comprises:
providing a film of a thermoplastic material in contact with a nanostructured surface of a flexible imprint stamp between a pair of molds;
forcing the molds together at an elevated process temperature, wherein at least one semi-cylindrical gutter portion is formed in the film by the molds and wherein a nanostructure pattern of the imprint stamp is imprinted in a surface of the gutter portion;
joining two gutter portions to form the tube, having the imprinted nanostructure pattern on an inner surface of the tube.

According to another aspect, the proposed solution provides an apparatus comprising a molding device having a pair of facing molds, wherein the molding device is configured to force the molds together at an elevated process temperature to form, in a film of a thermoplastic material arranged in contact with a nanostructured surface of a flexible imprint stamp between the pair of molds, at least one semi-cylindrical gutter portion, wherein two gutter portions are joinable to form a tube suitable for medical use having the imprinted nanostructure pattern on an inner surface of the tube.

Based on the proposed solution, methods and devices are obtained for producing a tube suitable for medical treatment purposes, which counteracts microorganisms from sticking to tube surfaces, based on the use of structural surface elements obtained by the nanoimprint to exert mechanical pressure on bacterial cell walls.

### Brief description of the drawings

The proposed solution and various embodiments thereof will be described in more detail below with reference to the accompanying drawings.
Fig. 1 schematically illustrates a tube having a nanoimprinted structured inner surface, according to an example of the proposed solution.
Fig. 2 schematically illustrates a flexible imprint stamp, including a cross-sectional view of a structured surface of the flexible imprint stamp, which may be used in the production of the tube in an example of the proposed solution.
Fig. 3A schematically illustrates an apparatus for producing a tube suitable for medical purposes, according to an example of the proposed solution.
Fig. 3B schematically illustrates a molding device of the apparatus for producing a tube suitable for medical purposes, according to an example of the proposed solution.
Fig. 4 schematically illustrates a pair of molds which may be used in the production of a tube suitable for medical purposes, according to an example of the proposed solution.
Fig. 5 schematically illustrates the pair of molds in mutual alignment for producing tube halves, according to an example of the proposed solution.
Fig. 6 is a flowchart of steps which may be included in a method for producing a tube suitable for medical purposes, according to an example of the proposed solution.
Fig. 7A schematically illustrates a loading stage of the pair of molds in mutual alignment with a flexible imprint stamp and a film sandwiched between the molds, according to an example of the proposed solution.
Fig. 7B schematically illustrates a molding or shaping stage at which gutter portions are formed in the film with at least one nanostructure surface imprint, according to an example of the proposed solution.
Fig. 7C schematically illustrates separation of the flexible imprint stamp from the shaped film, according to an example of the proposed solution.
Fig. 7D schematically illustrates cut-out gutter portions from the shaped film, forming tube halves, according to an example of the proposed solution.
Fig. 7E schematically illustrates joined tube halves to obtain tubes, according to an example of the proposed solution.
Fig. 8 schematically illustrates a cross-sectional view of a tube with a nanostructure imprinted on its inner surface, and joint arranged outwardly of the imprinted nanostructures, according to an example of the proposed solution.
Fig. 9A shows an image taken of a nanoimprinted surface, according to an example of the proposed solution.
Fig. 9B shows an image taken of a film of a thermoplastic material molded to shape semicylindrical gutter portions in which a nanoimprinted surface is formed, according to an example of the proposed solution.

### Detailed description

Embodiments of the present invention will hereinafter be described in more details with reference to the accompanying drawings. This invention may, however, be embodied in many different forms and should not be construed as limited to the embodiments set forth herein. Rather, these embodiments are provided so that this disclosure will be thorough and complete and will fully convey the scope of the invention to those skilled in the art. Unless otherwise defined, all terms (including technical and scientific terms) used herein have the same meaning as commonly understood by one of ordinary skill in the art to which this invention belongs. It will be further understood that terms used herein should be interpreted as having a meaning that is consistent with their meaning in the context of this specification and the relevant art and will not be interpreted in an idealized or overly formal sense unless expressly so defined herein. The terminology used herein is for describing particular embodiments only and it is not intended to be limiting of the invention. As used herein, the singular forms "a", "an" and "the" are intended to include the plural forms as well, unless the context clearly indicates otherwise. It will be further understood that the terms "comprises" "comprising," "includes" and/or "including" and the like, when used herein, specify the presence of stated features, integers, steps, operations, elements, and/or components, but do not preclude the presence or addition of one or more other features, integers, steps, operations, elements, components, and/or groups thereof.

The proposed solutions as outlined herein relates to the field of tubes for medical purposes, specifically to tubes intended to be inserted into the human body of the purpose injecting and/or withdrawing fluid. In some examples the tube is configured to be used as an indwelling catheter.

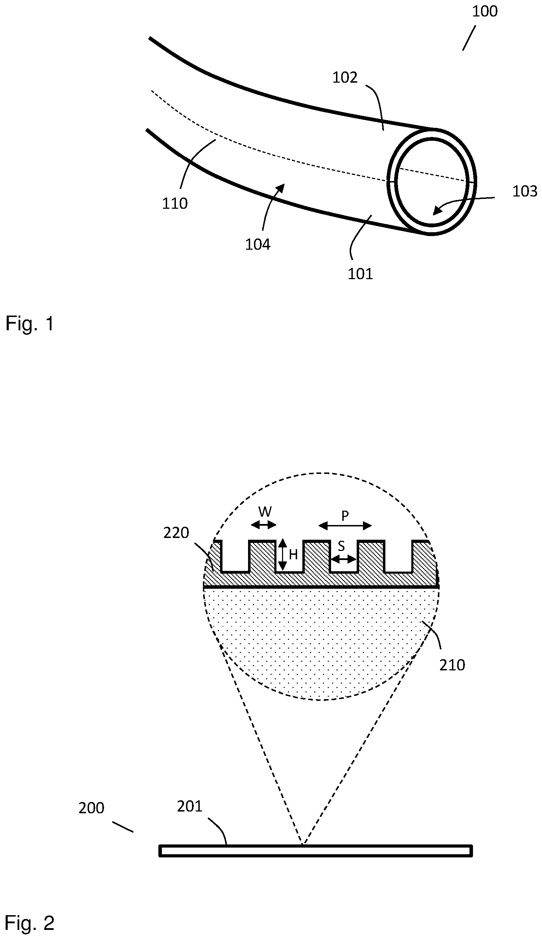

Fig. 1 shows a part of a tube 100 according to the proposed solution. The tube is composed of a number of tube portions, which are joined to form the tube shape. Reference will predominantly be made herein to examples where the tube portions are semi-cylindrical, as shown in the drawing, such that two tube portions 101, 102 are joined to form a cylindrical tube 100. In other examples, three, four or more tube portions may instead be joined to the form the tube 100.

The tube portions 101, 102 are gutter-shaped, and engaged with each other at a joint 110 to form the tube 100. Each tube portion 101, 102 has concave surface and a convex outer surface. The tube portions 101, 102 are in some examples manufactured in a common process, or in separate but repeated steps of the same process and are thus substantially identical. When the tube portions are joined, the concave surfaces form an inner surface 103 of the tube 100, whereas the convex surfaces form an outer surface 104 of the tube 100.

Form the purpose of mitigating or preventing biofilm formation on one or both of the surfaces 103, 104, specifically the inner surface 103, the tube comprises a nanostructure pattern, imprinted in each tube portion 101, 102. It has been found that nanoimprint technology is particularly suitable for use for manufacturing tubes 100 having surfaces configured to prevent biofilm development. Specifically, the formation of the gutter-shaped tube parts and the creation of the nanostructure pattern in the surface(s) can be made with excellent quality in a common process. Further aspects related to the manufacturing process for producing the tube 100 will now be described with reference to the drawings.

Initially, imprint lithography will be described. Imprint lithography is a technique for surface pattern replication, which is particularly suitable for transfer of small-scale patterns. The technique involves a process for transferring a pattern from a template, also commonly referred to as a stamp, to an object by contacting a structured surface of the stamp with a moldable layer on a target surface of the object. The moldable layer is subsequently processed to harden, or cure, and once the template or stamp has been removed from the object, an inverse replica is formed in the target surface. Imprint technology has proven to be one of the most powerful techniques for reproducing nano structures, i.e. structures below 1 µm such as in the order of 100 nm or smaller and is therefore generally referred to as nanoimprint lithography (NIL) when operated for transfer of patterns at such a scale. Specifically, NIL is advantageous for pattern transfer of large surfaces, such as up to 500x500 mm or larger.

A master template in which the pattern is formed must be produced with high accuracy, since any error in the master template is transferred to its replicas. The master template moreover needs to be durable, so to be able to be used repeatedly. A delicate process which may cause damage to the master template, or to the imprinted object, is the step of disassembling the two after the imprint step. The instant applicant was therefore one of the pioneers in developing a technique for transferring micro and nano scale patterns from master templates to produce final product substrates by using a two-step process of imprinting. This has inter alia been described in EP1959299A2. In a first step, an Intermediate Polymer Stamp (IPS) is created by imprint of the master template into a polymer foil. After separating the IPS from the master template, the IPS is subsequently used for transferring its imprinted pattern to final substrates in a second imprint step. Where the substrate is a rigid element, such as e.g. a silicon wafer,, imprint process typically involves the application of a moldable layer, commonly referred to as a resist, onto the substrate. The patterned surface of the IPS is then pressed into the resist layer to transfer the desired pattern. One advantage with such a process is that the IPS is flexible and can thus be disassembled from the master template in the first step, and from the imprinted substate in the second step, with minimized risk for causing damage to the master template and to pattern imprinted in the resist on the substrate.

The imprint process is thus a technique involving contact and disassembly. Moreover, the process may in various cases involve application of heat and/or radiation, for making a surface layer moldable and subsequently hardening the structures imprinted in the moldable layer. All these parameters and requirements place constraints on the possible materials that may be employed. This is particularly the case for a two-step imprint process, wherein IPS needs to be provided using materials which are suitable for use with both the master template and the target substrate.

Fig. 2 schematically illustrates a stamp 200, having a structured stamp surface 201. The stamp 200 is formed as a thin flexible sheet, such as with at least a body 210 formed of a polymer foil. The stamp 200 is also referred to herein as a flexible imprint stamp or an IPS. The stamp may be in the range of 5-500 µm thick, such as 100-250 µm. This ensures proper function upon imprinting, i.e., inversely replicating, the nano-sized pattern arranged in a surface layer 220 of the stamp 200 onto a surface of a tube having a diameter in the mm range, as will be described in more detail below. A cross-section of a magnified portion of the stamp 200 is shown at the top of the drawing, indicating a part of the structures identifying a three-dimensional pattern in the surface 201. The structures may include a variety of shapes, and the shown portion is merely an example, having a pattern defined by one or more of heights/depth H, pitch P, width W (e.g., diameter d), and spacing S. As noted, in the field of nanoimprint lithography, these parameters may be in the nanometer region, such as being smaller than 1 micrometer. The stamp 200 may be provided with the structures in the layer 220, provided on top of the body portion 210. The layer 220 may be a separately coated film on top of a base or substrate 210, where the base 210 and the layer 220 may be of different materials. Alternatively, the layer 220 may simply be a portion of a single material stamp 200, where the layer 220 is defined as a portion stretching from a surface of the object down to a certain depth into the bulk of the stamp 200. The structures may in various embodiments be formed by heating up the stamp 200 above a glass transition temperature T_{g} of the polymer material of the layer 220, after which a template is imprinted into the thus moldable layer 220 to imprint the pattern, followed by cooling down to below said glass transition temperature during the imprinting. This may be referred to as a hot embossing process. Alternatively, or additionally, the polymer of the layer 220 may be cured or cross-linked with the help of UV-light exposure during the imprinting process. A stamp 200 formed of a polymer foil may thus be provided as a flexible and optionally transparent foil comprising a thermoplastic polymer, a thermosetting polymer, and/or a polymer or pre-polymer which is cross-linked upon exposure to radiation, such as acrylic copolymers blended with UV curable oligomers. In some examples, the stamp may be obtained using a process according to the aforementioned patent application.

In accordance with the solutions proposed herein, a flexible imprint stamp 200 is suitable for use in the production of the tube 100 to obtain the imprinted nanostructure pattern on its inner surface 103. A tube fabrication process is described below, in which the nanostructure is imprinted in a film while molding the film into semi-cylindrical gutter portions.

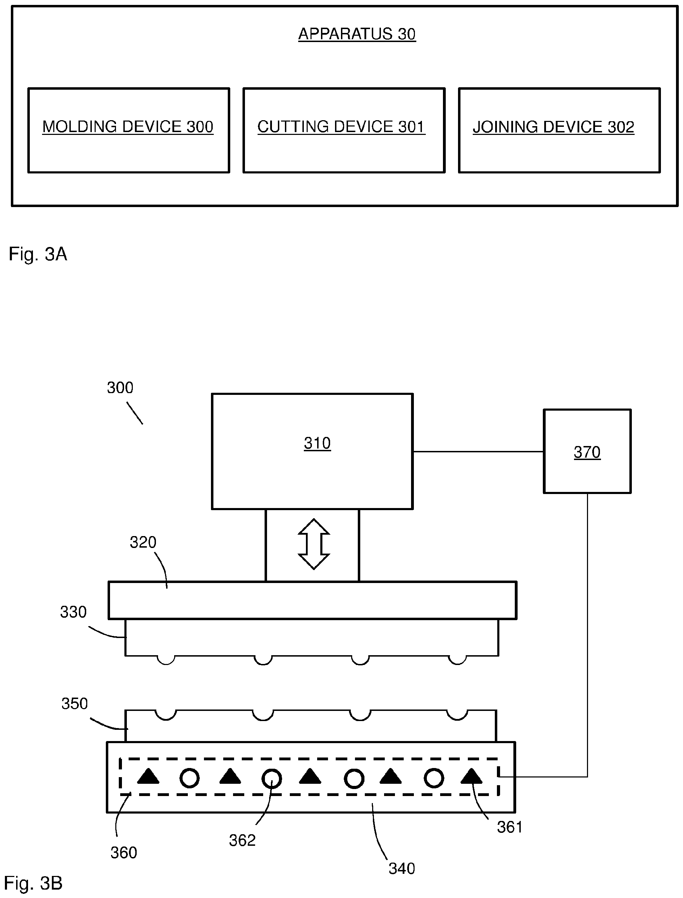

Fig. 3A schematically illustrates an apparatus 30 useable for production of a tube 100 with an imprinted inner surface, according to the proposed solution. The apparatus comprises a molding device 300 for shaping and imprinting tube portions 101, 102. The apparatus may further comprise a cutting device for cutting out shaped tube portions. The apparatus may further comprise a joining device 302, configured to combine and join tube halves to form the tube 100.

Fig. 3B schematically illustrates an example of a molding device 300, which may be or form part of an apparatus useable for production of a tube 100 with an imprinted inner surface, according to the proposed solution.

The molding device 300 is configured with a pair of facing molds 330 and 350. The molds 330, 350 may be permanently affixed to face each other in the molding device 300. Alternatively, one or two mold connectors 320 and 340 are included in the molding device, which are configured to selectively hold and optionally align the molds 330, 350. The molds 330, 350 may in such an example be held mechanically, e.g., by screws or clamps by the mold connector 320, 340, and may be properly aligned to each other, e.g., by guiding pins at the respective interface 320-330 and/or 340-350 between the mold connector and the mold.

A control unit 370 is configured to control a molding process, which may include control of force or pressure accomplish in the molding process, process temperature, and timing of various sub-steps of the molding process. In some examples, the control unit 370 comprises logic circuitry which may include a processing device, including one or multiple processors, microprocessors, data processors, co-processors, and/or some other type of component that interprets and/or executes instructions and/or data. The processing device may be implemented as hardware (e.g., a microprocessor, etc.) or a combination of hardware and software (e.g., a system-on-chip (SoC), an application-specific integrated circuit (ASIC), etc.). The processing device may be configured to perform one or multiple operations based on an operating system and/or various applications or programs. The control unit 370 may further include memory storage, which may include one or multiple memories and/or one or multiple other types of storage media. For example, the memory storage may include a random-access memory (RAM), a dynamic random-access memory (DRAM), a cache, a read only memory (ROM), a programmable read only memory (PROM), flash memory, and/or some other type of memory. The memory storage may be configured for holding computer program code, which may be executed by the processing device, wherein the control unit 370 is configured to control the molding device 300 to carry out any of the method steps as provided herein. Software defined by said computer program code may include an application or a program that provides a function and/or a process. The software may include device firmware, an operating system (OS), or a variety of applications that may execute in the logic.

In some examples, one mold may be aligned with the facing mold by a motor mechanism (not shown), such as an electric step engine, configured to change lateral configuration between the molds 330, 350 to obtain proper alignment. The motor mechanism may be controlled by the control unit 370, and may use alignment sensors (not shown), such as optical sensors, to determine alignment.

A pressure unit 310 is included in the molding device, configured to displace at least one of the molds 330, 350 to move them closer to or farther away from each other, as indicated by the arrow. The pressure unit may be configured to apply a force to pressure the molds 330, 350 together, e.g., by hydraulic or pneumatic pressure. The pressure unit 310 may be controlled and optionally powered by the control unit 370.

The molding device further comprises a heater unit 360, configured to supply heat to at least one of the molds 330, 350, or optionally directly to both molds 330, 350. While the drawing shows the heater unit as being comprised in the mold connector 340, it shall be understood that the heater unit 360 may comprise members arranged in one or both mold connectors 320, 340 and/or directly in one or both molds 330, 350 themselves. The heater unit 360 may comprise heater members, e.g., electric wires, connectable to the control unit 370, The heater unit 360 may further comprise cooling members 362, such as ducts formed in one or both mold connectors 320, 340 and/or directly in one or both molds 330, 350 themselves, through which ducts a cooling fluid is passed under control of the control unit 370. The heater unit 360 may further comprise on or more temperature sensors (not shown), from which measurement data may be supplied to the control unit 370, for proper temperature control to accomplish a desired supply of heat or to reach a certain process temperature, by supply of energy to the heater members 361 or cooling medium (e.g., flow of a cooling fluid) to the cooling members 362.

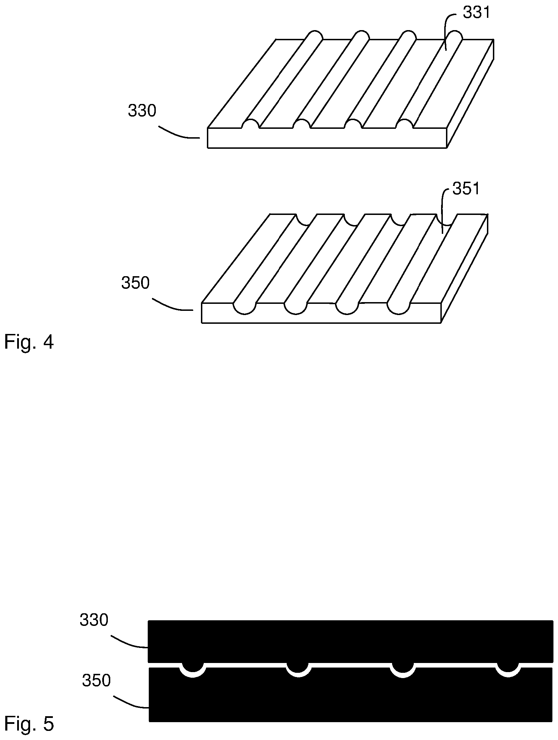

Fig. 4 schematically illustrates a pair of molds according to the proposed solution, as also included in the molding device of Fig. 3B. Here, the molds 330, 350 are presented with their respective mold structures facing upwards, for reasons of visibility. As illustrated in Fig. 3B, though, they will be used in the molding device 300 with the mold structures facing each other.

A first mold 330 has a mold structure comprising at least one ridge 331, which forms a mold structure configured to mold an inner tube surface 103. A second, mating, mold 350 has a mold structure comprising at least one groove 351, which forms a mold structure configured to mold an outer tube surface 104. In one example, the molds 330, 350 are manufactured in aluminum, though other metals may alternatively be used.

In some embodiments, as illustrated, a plurality of ridges 331 may be formed on the surface of the first mold 330, with a plurality of mating grooves 351 formed on the second mold 350, which are configured to cooperate to form a substantially semicylindrical spacing between them for formation of tube portions. Having a plurality of ridges 331 grooves 351 allows for multiple tube portions to be manufactured in a single mold stage. This provides for reduced production time and effort. In one example, the ridge 331 and the groove 351 run from side to side of the respective mold 330, 350.

Fig. 5 illustrates the pair of molds 330, 350 corresponding to the example of Fig. 4, arranged close to each other with mating mold structures. In the shown example, the ridge 331 and the groove 351 are configured to mold a semicircular shape in a film. For this reason, the ridge 331 may substantially have a semicircular shape in cross-section, so as to form the inner tube surface 103. In this context, semicircular shape is intended to also comprise near semicircular shapes, such as half oval. The groove 351, configured to form the outer surface 104 of the tube, may in some examples have a cross-section which is slightly less than semicircular, i.e., sub-semicircular, such that they together leave a semicircular gap, as shown in the drawing. The cross-section of the ridge 351 is thus configured dependent on the intended inner and outer diameter of the tube 100 to be formed, and also on the combined thickness of the film and the flexible stamp (which are not shown in Fig. 5). Although not shown in the drawing, the edges where the semicircular cross-section meets the planar surface of the respective mold 330, 350 may in some examples be rounded to ease separation of the stamp 200 from the formed and imprinted film and to minimize the risk of damaging the flexible imprint stamp 200.

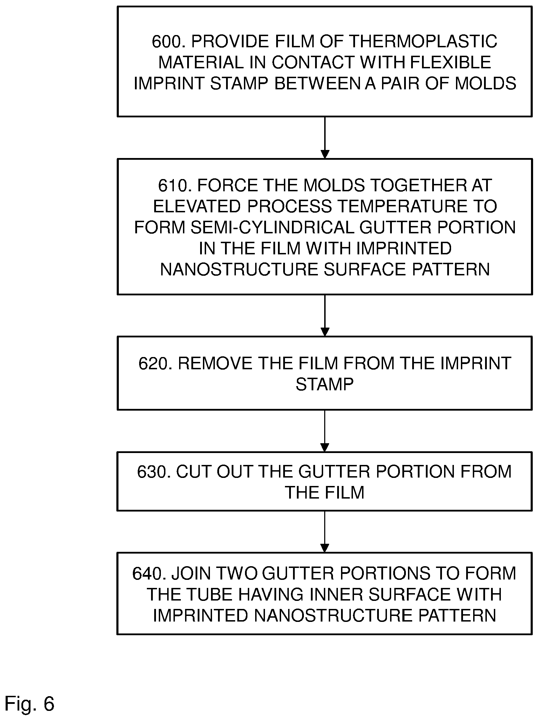

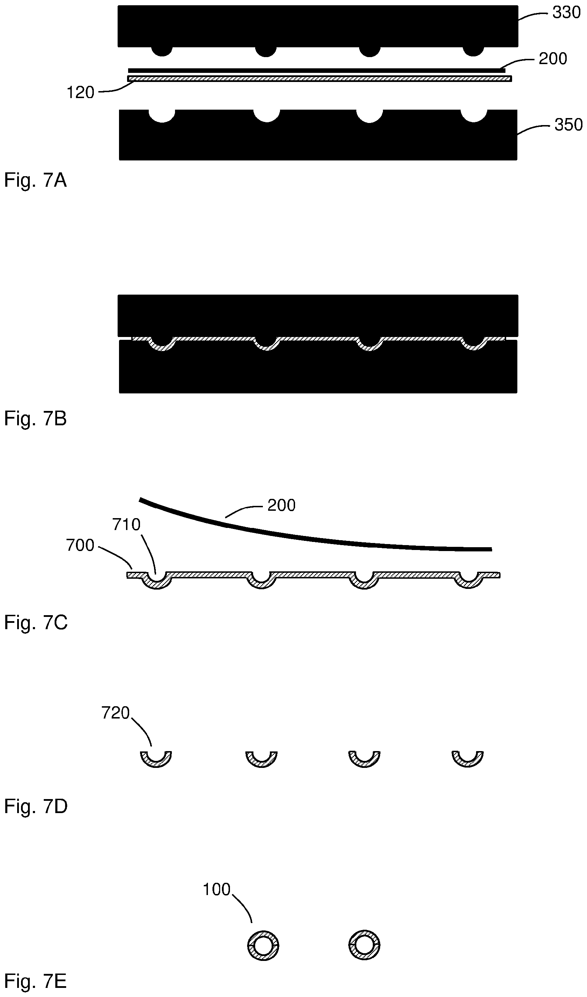

A method of producing a tube 100 suitable for medical use will now be broadly described with reference to Fig. 6, which method may be employed using the molding device 300. Details and examples related to the method are further described with reference to Fig. 7. For ease of understanding, reference is made to the elements already described with reference to Figs 1-5, by way of example.

In step 600, a film 120 of a thermoplastic material is provided in contact with a nanostructured surface 201 of a flexible imprint stamp 200 between a pair of molds 330, 350. This is also shown in Fig. 7A, where the film 120 and the stamp 200 are in separated arrangement for improved visualization. In some examples, the film 120 may be a sheet, placed in contact with the imprint stamp 200. In some examples, the film 120 may be provided from a roll. In other examples, the film may be spin-coated, sprayed, or otherwise provided in substantially liquid form onto the imprint stamp 200 or on one of the molds 330, 350 (typically the lower mold), or as powder or pellets which are melted in the molding device.

In various examples, the film 120 is made of thermoplastic urethane (TPU), which has been tested in the proposed method with good results, such when as using available TPU brand Platilon^{®} U2102N. For the purpose of producing tubes 100 of 3 mm outer diameter, the film 120 may in various examples have an initial thickness of 300-500µm.

The flexible imprint stamp 200, as described with reference to Fig. 2, may in some examples be a 300 µm thick flexible polymer, Fluon ETFE, from Asahi Glass Co.

In step 610, pressure is applied to force the molds 330, 350 together at an elevated process temperature. This may be carried out under control of the control device 370, wherein the pressure unit 310 is controlled to supply the force to press the molds 330, 350 together, and wherein the heater device 360 is controlled to obtain the desired process temperature. In this process, at least one semi-cylindrical gutter portion is formed in the film 120 by the molds and wherein a nanostructure pattern of the imprint stamp 200 is imprinted in a surface 103 of the gutter portion. This is also shown in Fig. 7B. In some examples, the molds 330, 350 may be controlled to obtain a spacing of 600 µm.

In one example which has been used, the step 610 was carried out with the following process steps 1-3 of Table 1.

**Table 1.**

| Step | Time (s) | Pressure (bar) | Temperature (°C) |
|---|---|---|---|
| 1 | 180 | 30 | 140 |
| 2 | 300 | 50 | 165 |
| 3 | 180 | 50 | 140 |
| Demolding | 60 | 40 | 50 |

With reference to Table 1, this example of the molding step 610, i.e., applying pressure to force the molds 330, 350 together at an elevated process temperature, is further explained below and may be carried out as follows:
Each step in Table 1 may represent a certain state obtained in the molding device 300, characterized by certain process parameters (at least pressure and temperature), and the time those process parameters are to be maintained. By way of example, in step 1, the pressure will be increased immediately to the set pressure (30 bar) to fixate the surface of the flexible stamp 200 against the film 120, whereafter the temperature starts to be increased. Once the set temperature of the process parameter is reached (140°C), a timer starts which runs the time set (180s) for maintaining that state. After that, steps 2 and 3 are carried out in comparable manner, using the respective process parameters, at which the gutter portions are formed, and the surface imprint is obtained.

During the demolding step, temperature will be brought down (to 50°C) for thermosetting, while keeping a defined pressure (40 bar) above atmosphere pressure. The defined pressure and temperature is maintained for 60s, after which the molds 330, 350 may be separated.

It should be noted that this example of process parameters, in particularly the time, only provides examples that have been successfully used. Other process characteristics are plausible, dependent on, inter alia, material and thickness of the film 120.

In step 620, the imprint stamp 200 is removed from the film, which has now been shaped by molding to display at least one gutter portion. The separation may be carried out after cooling down and releasing the pressure, by any known method such as peeling. Fig. 7C shows the now shaped and separated film 700 comprising gutter portions 710.

In step 630, the gutter portions 710 may be cut out from the film 700, prior to joining the gutter portions 710. This is also shown in Fig. 7D, wherein individual semicylindrical tube members 720 are obtained. These correspond to the tube halves 101, 102 of Fig. 1. The cutting may be carried out by any suitable method, such as by mechanical edge cutting or laser cutting. In one example, cutting is carried out laterally, i.e., in the plane of the film 700. In another example, the cutting may be carried out vertically, i.e., perpendicular to the plane of the film 700. The cutting may be executed in the cutting device 301.

In step 640, two gutter portions 710 are joined to form the tube 100, with the imprinted nanostructure pattern from the stamp 200 imprinted on the inner surface 103 of the tube 100. In the joining process, opposite tube parts 101, 102 may be aligned with each other by placing them to encompass an alignment rod (not shown) in the channel of the tube 100 to be formed. The joining may be carried out by microwave welding, plasma welding, thermal knife, laser welding, or other suitable method used for joining pieces of thermoplastic material. Tubes 100 thereby formed are further illustrated in Fig. 7E. The joining may be executed in the joining device 302. The joining may be arranged to adhere engaging outer parts of the two gutter portions 101, 102 without melting inner parts of the gutter portions. This may result in an outwardly located joint, such as a cured melt of the film material, which does not appear at the imprinted inner surface 103.

It may be noted that in an alternative embodiment, the cutting out of the gutter portions 710 may be carried out after joining two shaped films 700 with their formed gutter portions 710 facing each other. In other words, step 640 may be carried out prior to step 630.

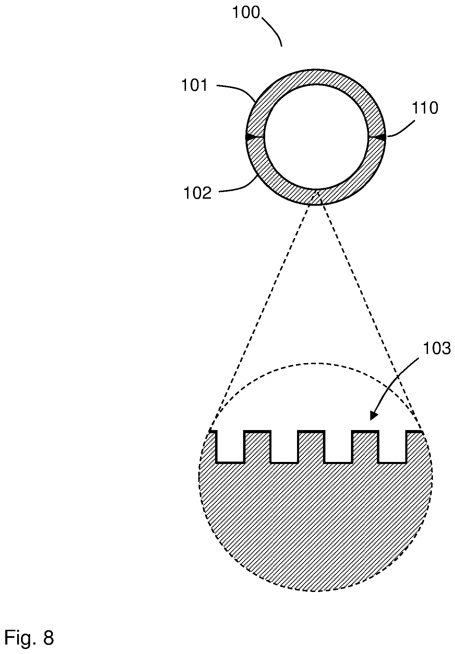

Fig. 8 shows a larger cross-sectional view of the tube 100, obtained by means of an example of the proposed method. This drawing also indicates the obtained nanostructured pattern on the inner surface 103 of the tube, in enhancement. It may be noted that the apparatus and process described with reference to Figs 6 and 7 may additionally, or instead, be employed for imprinting the outer surface 104 of the tube. This may be obtained by providing the imprint stamp 200, additionally or optionally, on the side of the film 120 facing the mold 350 configured with the grooves 351.

As already noted, in the context of joining the semicylindrical tube members 720 it is advantageous that the inner part of the tube 100 shall not be melted so that the imprinted pattern, which is intended to be bactericidal, gets eliminated or damaged in the joint 110. Therefor the depth of the melting is preferably controlled to be smaller than the thickness of the tube wall. The joining is thus controlled so that the resulting joint 110 is a located to adhere outer parts of the gutter portions 720 and extends inwards of the tube 100 to a position outwardly of the nanostructure pattern on the inner surface 103 of the tube 100, as shown in Fig. 8. In some examples, this is obtained by controlling the supplied energy (microwave, laser, plasma, etc.) for obtaining the joint 110, or the penetration depth of a melting knife. In some examples, the joining is carried out by the joining device 302, configured to adhere two gutter portions to form the tube. As noted, the joining may be carried out after or before cutting out the semicylindrical tube halves 720, 101, 102.

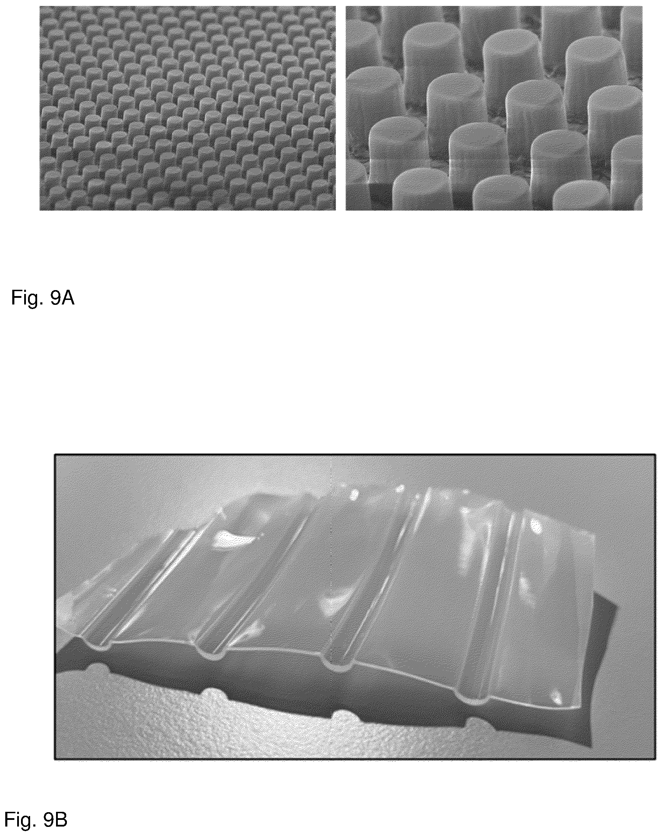

Fig. 9A shows an image captured by Scanning Electron Microscopy (SEM) of an imprinted pattern on the inner surface 103 of the tube 100, as obtained using the proposed solution. The nanostructure pattern may comprise nanostructures with a projection in a range of 80-300 nm. In some examples, the nanostructures may have a pitch in a range of 400-500 nm. In some examples, said nanostructures have a width in a range of 200-350 nm. Nanoimprint provides the benefit of a wide selection of shapes and sizes of the nanostructures to suit the intended need, so as to obtain effective prevention of biofilm growth. The imprint stamp 200 may thus be suitably designed to accomplish the intended pattern on the tube 100. Table 2 below shows shape and size of the nanostructures in two examples of nanoimprint lithography (NIL) patterns on TPU, which have been tested by the applicant.

**Table 2.**

| NIL pattern | Height H (nm) | Pitch P (nm) | Diameter d (nm) | Spacing S (nm) | Aspect ratio (H/d) |
|---|---|---|---|---|---|
| NP2 | 214 ±2 | 414 ±28 | 108 ±14 | 306 ±31 | ~2 |
| NP1 | 162 ±6 | 447 ±11 | 209 ±10 | 238 ±15 | ~1 |

Fig. 9B shows a picture of the film 700 comprising gutter portions 710, as obtained using the proposed solution. The gutter portions 710 are configured as semicircular elements, with an outer diameter of 3 mm. Other tube diameters are conceivable, though, which will be defined by the mold structures on the molds 330, 350, such as diameters between 2-9 mm.

The tube 100 according to the proposed solution, as well as the apparatus and method for producing the tube, has been described in the foregoing. The proposed solution is particularly suitable for obtaining a tube suitable for medical treatment purposes, such as a tube intended to be inserted into the human (or animal) body, e.g., a catheter. The proposed solution provides for the tube 100 having a nanostructured pattern on at least the inner surface 103. These surfaces can prevent or mitigate biofilm development. This in turn can decrease reduce the antibiotic drugs intake and hence lower the risks of AMR.

The production solution includes cost-effective flexible polymer substrate 200 NIL patterning to obtain a tube surface exhibiting bactericidal characteristics. The use of the flexible polymer stamp 200 is advantageous over the plausible alternative of using molds which are preconfigured with the imprint pattern defined on the surfaces of the ridges and grooves. In such a configuration, demolding of the film from the molds would be very challenging, because of stronger adhesion between the solid stamp and the film.

The proposed solution is defined by the terms of the appended claims. Various examples of the proposed solution have been outlined in the foregoing and may include any combination of the items set out below.

Item 1. A method for producing a tube suitable for medical use, comprising:
providing (600) a film of a thermoplastic material in contact with a nanostructured surface of a flexible imprint stamp between a pair of molds;
forcing (610) the molds together at an elevated process temperature, wherein at least one semi-cylindrical gutter portion is formed in the film by the molds and wherein a nanostructure pattern of the imprint stamp is imprinted in a surface of the gutter portion;
removing (620) the imprint stamp from the film;
joining (640) two gutter portions to form the tube, having the imprinted nanostructure pattern on an inner surface of the tube.

Item 2. The method of item 1, wherein the thermoplastic material is thermoplastic urethane.

Item 3. The method of item 1 or 2, wherein said pair of molds comprises mating mold structures, including at least one ridge (331) on one of said molds and at least one groove (351) on the other mold, which cooperate to form said gutter portion.

Item 4. The method of any preceding item, wherein the molds are configured to obtain an outer diameter of the tube in a range of 2-9 mm.

Item 5. The method of any preceding item, wherein the processing temperature is in a range of 130-170 degrees Celsius.

Item 6. The method of any preceding item, wherein said pattern comprises nanostructures with a projection in a range of 80-300 nm.

Item 7. The method of item 6, wherein said nanostructures have a pitch in a range of 400-500 nm.

Item 8. The method of item 6 or 7, wherein said nanostructures have a width in a range of 200-350 nm.

Item 9. The method of any preceding item, comprising:
cutting (630) out the gutter portion from the film.

Item 10. The method of any preceding item, wherein the joining comprises adhering engaging outer parts of the gutter portions without melting inner parts of the gutter portions.

Item 11. The method of any preceding item, wherein the joining comprises microwave welding at an interface engaging the gutter portions.

Item 12. A tube (100) suitable for medical use, comprising two semi-cylindrical gutter-shaped tube portions (101, 102, of a thermoplastic material, engaged at a joint (110) to form the tube, wherein the tube comprises a nanostructure pattern on an inner surface (103) of the tube, imprinted in each tube portion.

Item 13. The tube of item 12, wherein the thermoplastic material is thermoplastic urethane.

Item 14. The tube of item 12 or 13, wherein the tube has an outer diameter in a range of 2-9 mm.

Item 15. The tube of any of items 12-14, wherein said pattern comprises nanostructures with a projection in a range of 80-300 nm.

Item 16. The tube of any of items 12-15, wherein said nanostructures have a pitch in a range of 400-500 nm.

Item 17. The tube of any of items 12-16, wherein said nanostructures have a width in a range of 200-350 nm.

Item 18. The tube of any of items 12-17, wherein the joint is located to adhere outer parts of the gutter portions and extends inwards of the tube to a position outwardly of the nanostructure pattern on the inner surface of the tube.

Item 19. An apparatus comprising a molding device (300) having a pair of facing molds (330, 350), wherein the molding device is configured to force the molds together at an elevated process temperature to form, in a film of a thermoplastic material arranged in contact with a nanostructured surface of a flexible imprint stamp between the pair of molds, at least one semi-cylindrical gutter portion (710), wherein two gutter portions are joinable to form a tube (100) suitable for medical use having the imprinted nanostructure pattern on an inner surface (103) of the tube.

Item 20. The apparatus of item 19, wherein said pair of molds comprises mating mold structures, including at least one ridge (331) on one of said molds and at least one groove (351) on the other mold, which are arranged to form said gutter portion in the film.

Item 21. The apparatus of item 19, wherein said pair of molds comprises mating mold structures, including a plurality of ridges (331) on one of said molds and a plurality of grooves (351) on the other mold, which are arranged to form a plurality of gutter portions in the film.

Item 22. The apparatus of any of items 19-21, wherein the molding device comprises a pressure device (310), a heating device (360), and a control unit (370) configured to control the pressure device to apply pressure to force the molds together at an elevated process temperature obtained by the heating device.

Item 23. The apparatus of any of items 19-22, wherein the molds are configured to obtain an outer diameter of the tube in a range of 2-9 mm.

Item 24. The apparatus of item 22 or 23, wherein the processing temperature is in a range of 130-170 degrees Celsius.

Item 25. The apparatus of any of items 19-24, comprising:
a cutting device (301), configured to cut out the formed gutter portion from the film.

Item 26. The apparatus of any of items 19-25, comprising:
a joining device (302), configured to adhere two gutter portions to form the tube.

Item 27. The apparatus of item 26, wherein the joining device is configured to adhere engaging outer parts of the two gutter portions without melting inner parts of the gutter portions.

Item 28. The apparatus of item 26, wherein the joining device comprises a microwave welding device.

## Claims

1. A tube (100) suitable for medical use, comprising two semi-cylindrical gutter-shaped tube portions (101, 102, of a thermoplastic material, engaged at a joint (110) to form the tube, wherein the tube comprises a nanostructure pattern on an inner surface (103) of the tube, imprinted in each tube portion.

2. The tube of claim 1, wherein the thermoplastic material is thermoplastic urethane.

3. The tube of claim 1 or 2, wherein the tube has an outer diameter in a range of 2-9 mm.

4. The tube of any preceding claim, wherein said pattern comprises nanostructures with a projection in a range of 80-300 nm.

5. The tube of any preceding claim, wherein said nanostructures have a pitch in a range of 400-500 nm.

6. The tube of any preceding claim, wherein said nanostructures have a width in a range of 200-350 nm.

7. The tube of any preceding claim, wherein the joint is located to adhere outer parts of the gutter portions and extends inwards of the tube to a position outwardly of the nanostructure pattern on the inner surface of the tube.

8. An apparatus comprising a molding device (300) having a pair of facing molds (330, 350), wherein the molding device is configured to force the molds together at an elevated process temperature to form, in a film of a thermoplastic material arranged in contact with a nanostructured surface of a flexible imprint stamp between the pair of molds, at least one semi-cylindrical gutter portion (710), wherein two gutter portions are joinable to form a tube (100) suitable for medical use having the imprinted nanostructure pattern on an inner surface (103) of the tube.

9. The apparatus of claim 8, wherein said pair of molds comprises mating mold structures, including at least one ridge (331) on one of said molds and at least one groove (351) on the other mold, which are arranged to form said gutter portion in the film.

10. A method for producing a tube suitable for medical use, comprising:
providing (600) a film of a thermoplastic material in contact with a nanostructured surface of a flexible imprint stamp between a pair of molds;
forcing (610) the molds together at an elevated process temperature, wherein at least one semi-cylindrical gutter portion is formed in the film by the molds and wherein a nanostructure pattern of the imprint stamp is imprinted in a surface of the gutter portion;
removing (620) the imprint stamp from the film;
joining (640) two gutter portions to form the tube, having the imprinted nanostructure pattern on an inner surface of the tube.

11. The method of claim 10, wherein the thermoplastic material is thermoplastic urethane.

12. The method of claim 10 or 11, wherein said pair of molds comprises mating mold structures, including at least one ridge (331) on one of said molds and at least one groove (351) on the other mold, which cooperate to form said gutter portion.

13. The method of any of claims 10-12, comprising:
cutting (630) out the gutter portion from the film.

14. The method of any of claims 10-13, wherein the joining comprises adhering engaging outer parts of the gutter portions without melting inner parts of the gutter portions.

15. The method of any of claims 10-14, wherein the joining comprises microwave welding at an interface engaging the gutter portions.
